# EUROPEAN PATENT SPECIFICATION

(11) **EP 4 457 511 B1**
(45) Date of publication and mention of the grant of the patent: **10.09.2025**
(21) Application number: 22844224.0
(22) Date of filing: 29.12.2022
(51) Int. Cl.: G01N 33/487, B01L 3/00, G01N 15/14, G01N 1/40, G01N 15/00

(54) **SELECTING FORCES FOR MEANS AND METHODS INVOLVING CELLULAR AVIDITY**
AUSWAHL VON KRÄFTEN FÜR MITTEL UND VERFAHREN MIT ZELLULÄRER AVIDITÄT
SÉLECTION DE FORCES POUR MOYENS ET PROCÉDÉS IMPLIQUANT UNE AVIDITÉ CELLULAIRE

(30) Priority: 31.12.2021 NL 2030378; 13.05.2022 EP 22173305
(43) Date of publication of application: 06.11.2024
(73) Proprietor: LUMICKS CA Holding B.V., 1105 AG Amsterdam (NL)
(72) Inventor: FASCI, Domenico, 1105 AG AMSTERDAM (NL); GREGG, Trillian Ashley, 1105 AG AMSTERDAM (NL); VAN LOENHOUT, Marinus Theodorus Johannes, 1105 AG AMSTERDAM (NL); DAVOLI, Serena Alba, 1105 AG AMSTERDAM (NL); DE GROOT, Mattijs, 1105 AG AMSTERDAM (NL)
(74) Representative: Algemeen Octrooi- en Merkenbureau B.V.
(86) International application number: PCT/EP2022/087998
(87) International publication number: WO 2023/126470

(56) References cited:
- WO-A1-2021/089654
- US-A1- 2021 364 439

## Description

### Introduction

In the art, binding studies have been conducted with target cells being attached to a plate, *e.g.* a glass plate, wherein subsequent cells of interest are added to the plate to interact (*e.g.* study the binding thereof) with the attached target cells. In particular, such studies can be performed to determine or assess cellular avidity, e.g. of effector cells such as CAR-T cells and cancer cells. Cellular avidity is than subsequently determined by exerting a force on the cells of interest and subsequently analysis of cells of interest that detach and/or remain attached to the target cells.

Patent application WO 2021/089654 A1 discloses a known prior art method for measuring a binding force of weakly and strongly bound effector cells from target cells bound to a surface substrate and being pulled by a force away from the substrate. A force ramp is applied to determine cell detachments and record two different avidity curves.

However, a problem that can be observed with target cells attached to a surface, is that in addition to specific binding of the cells of interest to the target cells, background binding, not necessarily attributable to specific binding, may play a role in the binding of cells to the target cells and/or surface. Such background binding, to which also can be referred to as aspecific binding, can be observed for example when control cells of the cells of interest are tested under the same conditions of binding studies of the cells of interest and show a substantial binding to the target cells attached to the surface. For example, when control cell binding is relatively high as compared with specific binding of cells of interest, when different cells of interest are to be compared it becomes much more difficult to differentiate to detect variation in cellular avidity.

Hence, there is a need in the art to provide for means and methods that can reduce binding of control cells of cells of interest to target cells attached to a surface, while at the same time allowing for effective binding of cells of interest to the target cells under the same conditions. This is highly useful when it is for example desirable to select for and identify candidate effector cells that can bind to target cells and/or modulate the cellular avidity of effector cells to target cells. This is in particular highly useful in methods in which cellular avidity is used as a means, *e.g.* by applying a force to cells of interest that interact with target cells. In such cellular avidity methods, it is highly desirable and important to have the ability to differentiate well between specific binding of cells of interest and background binding of control cells to target cells.

Hence, there is need in the art to provide for means and methods that may enlarge the window between positive and negative binding such that differentiation in cellular avidity between various cells of interest can be much improved, and/or cells of interest can be more efficiently identified.

### Summary of the invention

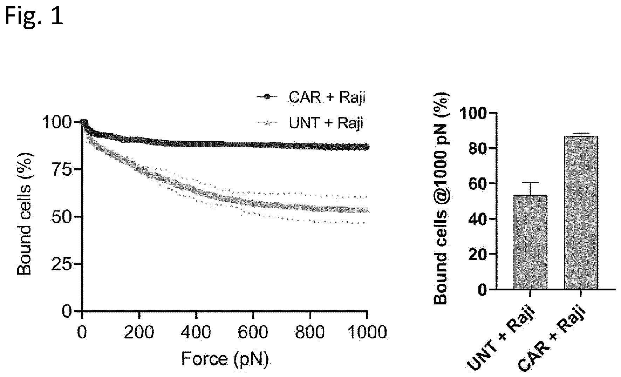

As said, when target cells are attached to a surface, and subsequently allowed to interact with control cells or cells of interest, *e.g.* with a receptor specific to the target cells, substantial background binding can confound specific cellular avidity, in particular in methods wherein cellular avidity is an important parameter. For example, the current inventors observed that the detachment of cells of interest and control cells, when applying a force thereon, sometimes results in a large portion of control cells to remain attached to the target cells (see *e.g.* Figure 1). Hence, when studying the binding of cells of interest to target cells by exerting a force on the cells of interest, such substantial background binding may confound the assessment and/or use of specific cellular avidity of (candidate) cells of interest with target cells (*e.g.* when selecting or sorting cells). This is because a large portion of the cells of interest that bind to target cells may be cells that bind aspecifically or at least cannot be differentiated therefrom, which is highly undesirable. Hence, the current inventors sought to improve, *i.e.* reduce, background binding in means and methods relying on cellular avidity.

Therefore, an object of the current invention is to provide for improved means and methods reducing observed background binding to target cells attached to a surface. As shown in the examples, it was surprisingly found that when different forces were applied to cells of interest and control cells thereof, after interacting with target cells for different defined periods, the percentage of control cells that remain attached may vary considerably, as opposed to the cells of interest which were shown to vary much less. Hence, this observation allows the selection of an applied force to improve, *i.e.* increase, the difference between the percentage of cells of interest and control cells. By varying the applied force and/or the force loading rate, the so-called window between negative and positive binding (*i.e.* background binding of control cells vs. binding of cells of interest, to target cells), can be improved which is highly advantageous in means and methods that rely on differentiating between cellular avidity of various cells of interest, and on ranking thereof. Hence, the current invention provides for means and methods in which different forces are applied, upon which a suitable force can be selected that allows for a much improved positive/negative window for subsequent cellular avidity methods.

### Figures

Figure 1. Aspecific binding in cellular avidity experiments: Untransduced (UNT, grey lines) or FMC63-transduced Jurkat (CAR, black lines) were layered on z-Movi^{®} chips coated with Poly-L-lysine (PLL), seeded with Raji cells and cellular avidity was measured. The left side displays the avidity curves where the y-axis indicates the percentage of bound cells, the x-axis indicates the force (pN). The right side displays the percentage of effector cells bound to the target cells at the end of the force ramp (1000 pN) as bar graphs. The data represents mean ± SD, n=2, and is representative of two independent experiments. In the graph on the left, mean is indicated as a solid line and the standard deviation of the experiments is indicated as dotted lines around the mean. Shown in the figure is that the aspecific/background binding of the untransduced control cells on the Raji cells (bottom) is relatively high. This leads to a small positive/negative window on the Raji cells (c.a. 50-85%) making it more difficult to detect differences in specific binding properties, *i.e.* cellular avidity, in the Raji setting.

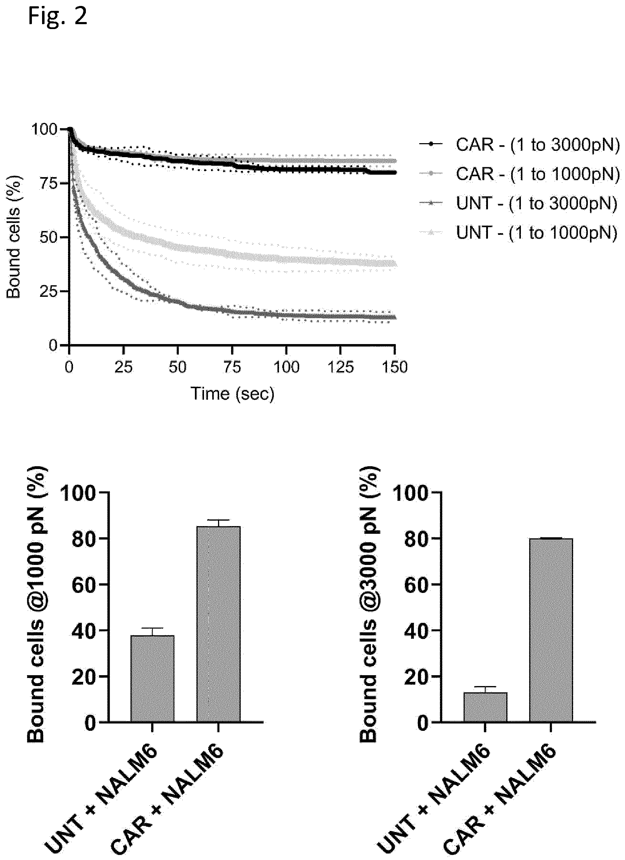

Figure 2. Increase on target end force reduces the aspecific binding on NALM6 in cellular avidity experiments: Cellular avidity of untransduced (UNT) or FMC63-transduced Jurkat (CAR) cells in NALM6-seeded z-Movi^{®} chips. The measurements were performed applying a force ramp of 2.5 minutes from 1 to 1000 pN or 1 to 3000 pN. A higher end force resulted in a drop of the aspecific binding (lower bottom lines, of which the lower line is from 1-3000 pN), while maintaining the binding of the positive (CAR) sample (upper two lines). The bottom panels display the percentage of cells bound at 1000 pN (left) or at 3000 pN (right), and highlight an increased and improved positive/negative window for samples measured with higher end force. The data represents mean ± SD, n=2, and is representative of two independent experiments.

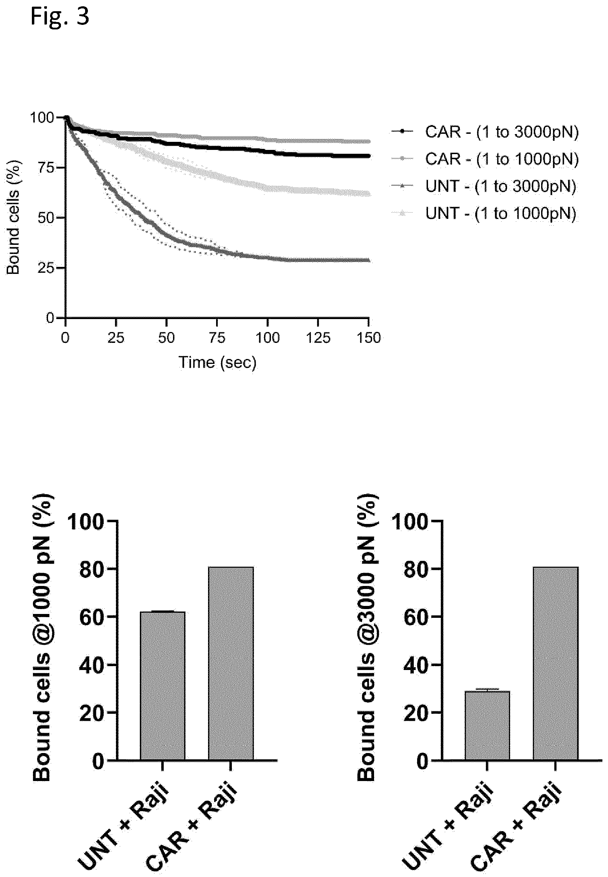

Figure 3. Increase on target end force improves the positive/negative window in cellular avidity experiments: Cellular avidity of untransduced (UNT) or FMC63-transduced Jurkat (CAR) cells in Raji-seeded z-Movi^{®} chips. The measurements were performed applying a force ramp of 2.5 minutes from 1 to 1000 pN or 1 to 3000pN. As observed in Figure 2, a higher end force resulted in a substantial improvement of the positive/negative assay window. The lower bottom lines represent untransduced cells, of which the lower line represents a force from 1-3000 pN which is substantially reduced as compared with the line above which represents 1-1000 pN, while the binding of the positive (CAR) samples were similar (upper two lines). The bottom panels display the percentage of cells bound at 1000 pN (left) or at 3000 pN (right). The data represents mean ± SD, n=1 or 2, and is representative of two independent experiments.

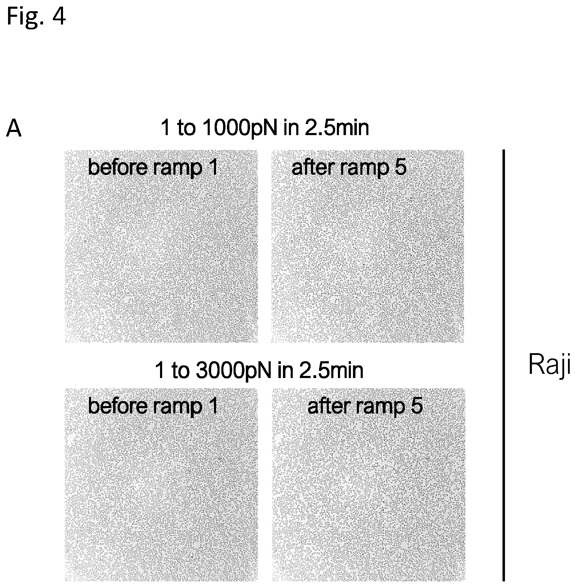

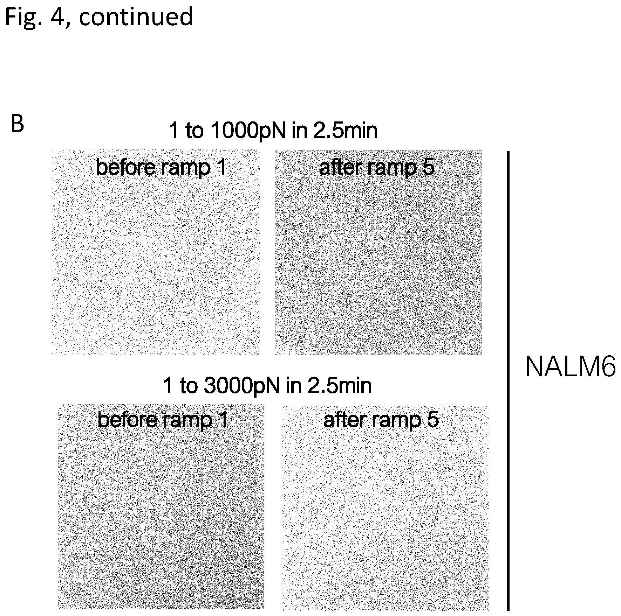

Figure 4. The NALM6 and Raji target cell monolayer viability and integrity is preserved after multiple force ramps to 3000 pN: z-Movi^{®} chips seeded with A) Raji or B) NALM6 cell monolayers were subjected to five force ramps of 2.5 minutes each to 1000 pN or to 3000 pN. The monolayer integrity was evaluated taking a snapshot before and after the force ramps, and viability was assessed qualitatively with Trypan Blue staining (right side snapshots). No differences in target cell monolayer confluency, cell morphology or staining could be detected indicating the target cells tolerated the procedure well. Data is representative of two independent experiments

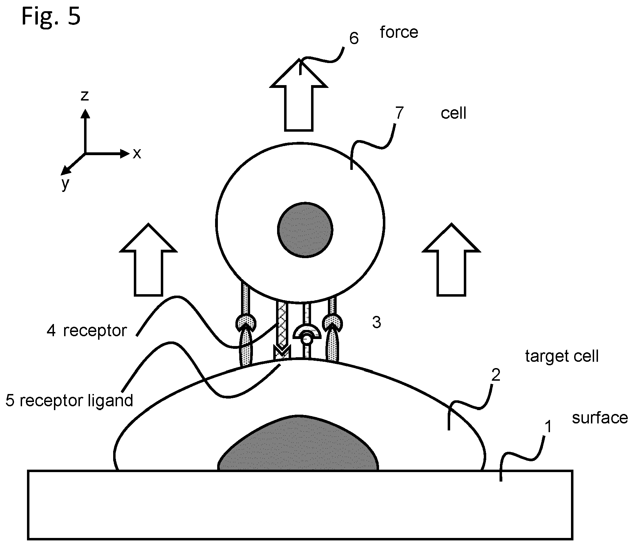

Figure 5. Schematic showing target cells and cells of interest and cellular avidity. Target cells (2) are provided on a surface (1), which in this case a flat surface. The target cell expresses ligands and receptors, likewise the cell (7) to be targeting the target expresses ligands and receptors as well (3). A specific ligand-receptor interaction (4 and 5) can be the driving force for the forming of a cell-cell bond with multiple ligand-receptor interactions combined resulting in strong cell-cell binding. To rupture this cell-cell bond, a force (6) is exerted on the cell (7) away from the target cell (2), which can be in the z-axis direction *e.g.* when the flat surface is defined as being in the x-y plane. Alternatively, this can also be in the x- or y-axis direction. When the cell-cell bond is ruptured, the cell moves away from the cell surface and/or the target cell and this event can be detected and/or detached cells can be collected and quantified and/or further analysed.

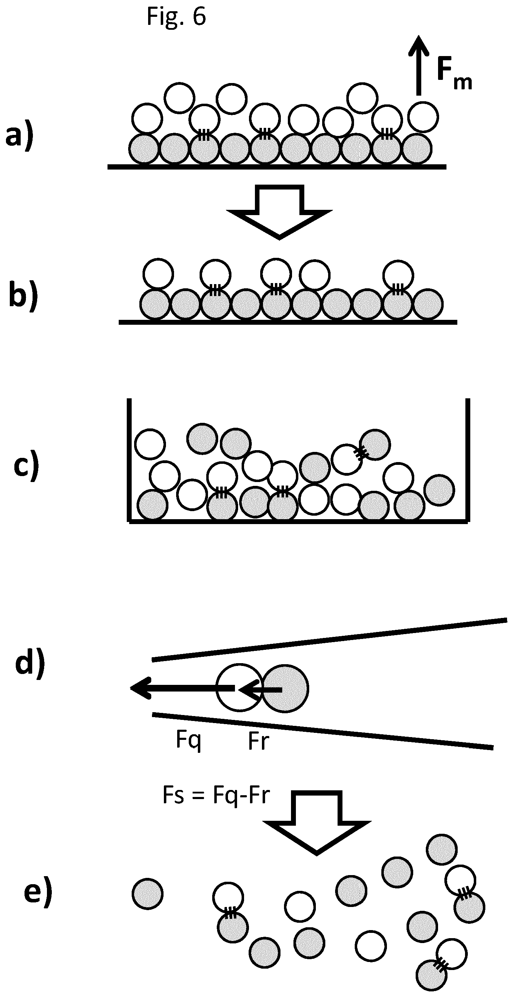

Figure 6. In a cellular avidity measurement with cells attached to a surface (depicted as grey cells), cells (depicted as white cells) are interacted with the attached cells to bind therewith, *e.g.* utilizing target cells expressing an antigen and cells of interest such as effector cells with a CAR against the antigen, as depicted in a). After a defined incubation, a force, Fₘ applied away from the attached cells. This results in cells detaching therefrom, either because they did not bind to the cells attached to the surface or because the binding strength was not strong enough, *e.g.* when aspecifically bound. Cells that remain were sufficiently strongly bound to the attached cells as depicted in b). Such a scenario is e.g. employed in the examples as described herein. Cells that formed a synapse may substantially remain (indicated with hashes). Alternatively, a cellular avidity measurement can be performed which does not require cells attached to a surface. In this scenario, *e.g.* target cells (depicted as white cells) are provided and cells of interest, *e.g.* effector cells (depicted as grey cells), and these are incubated for a defined period. After said incubation, cells are resuspended and a differential force, Fₙ, is applied. Such an applied force may be larger than typically used when cells are attached (Fₘ). This force may break cell-cell bonds, preferably aspecific cell-cell bonds as these bonds may be less strong when compared with specific cell-cell bonds such as e.g. synapse bonds, thereby resulting *e.g.* substantially specific cell-cell bonds. The cell suspension may be subsequently analysed with regard to singlets (either white or grey cells) and doublets (grey cell bound with white cell), which numbers may be used, *e.g.* to provide a cellular avidity score. Of course, one may also combine applying a differential force (shown in d), after a force has been applied, *e.g.* away from attached cells (in the scenario obtained as shown in b), or one may apply a differential force after incubation on attached cells (in the scenario as obtained in a). Cellular avidity scores may be determined by determining the number of grey cells and white cells bound to each other (doublets) and/or by counting the number of grey cells bound to white cells, *e.g.* effector cells bound to target cells, that formed a specific bond such as a synapse (doublets with hashes). Cellular avidity scores can be calculated taking into account initial amount of cells provided and/or single cells obtained in the methods. For example, by calculating the ratio of the number of cells of interest that remained bound to target cells after exerting the force to the number of cells of interest initially provided.

### Detailed description

WO 2018/083193 discloses a method, system and sample holder for manipulating and/or investigating cellular bodies; it makes use of acoustic forces generated by ultrasound standing waves in a microfluidic flow-cell. The acoustic force allows application of force to thousands of cells in parallel, for example, to thousands of T-cells in contact with a monolayer of cancer target cells. By detecting cell-cell rupture events (*e.g.* (CAR-)T cells releasing from a tumor cell monolayer) as a function of the acoustic force and/or by application of fluid flow in relation to an applied acoustic force, cell-cell binding interactions may be efficiently analysed and/or cells may be sorted according to cell-cell binding interactions. For example, an avidity curve can be generated by plotting the percentage of bound CAR T-cells as a function of the applied force (see *e.g.* Figure 1). Such a system, which is commercially available from LUMICKS and known as z-Movi^{®}, has been used in the example section as described herein. However, the means and methods in accordance with the invention as disclosed herein are not restricted to the use of acoustic force, but are of use with other types of forces that can be applied on cells, such as centrifugal/acceleration force or shear flow, and which can provide for similar graphs and plots as shown in the examples herein, all relying on the same inherent properties of cellular avidity. As long as a force can be applied and controlled on cells of interest that bind/interact with target cells attached to a surface, such a force is contemplated in accordance with the invention.

As said, a problem in means and methods involving cellular avidity and applying forces on cells of interest that interact with target cells that has been observed by the current inventors is that it can be difficult to differentiate between specific interactions and background binding. In accordance with the current invention, means and methods are provided that solve that problem, therewith providing for improvement in the capability to differentiate between specific interactions and background binding.

It is understood that "cellular avidity" as used throughout herein comprises the overall strength of interactions occurring in a cell-to-cell contact, involving a diversity of molecules at the surfaces of the cells that interact (see *e.g.* Figure 5). Such interactions may include a diversity of receptor-ligand pairs, among which *e.g.* a specific receptor-ligand interaction, occurring at the membrane surface of a cell. For example, when a T-cell receptor triggers the formation of an immune synapse by recognizing an antigen presented by an MHC molecule at an antigen presenting cell, the synapse formation involves such multitude of interactions, as also other membrane bound molecules are involved in the interactions (such as integrins and the like). Hence, "cellular avidity" may not be restricted to the interaction of *e.g.* the alpha and beta chain of the TCR and the antigen presented by MHC, but rather involves a multitude of interactions working jointly forming a strong bond between *e.g.* cells. It may also involve active signalling and processes internal to the cells such as *e.g.* during immune synapse formation. It is understood that the cellular avidity of a cell of a certain type is defined relative to the target cells and conditions tested.

Hence, in one embodiment, a method is provided of selecting a force to be applied, for differentiating between binding of cells of interest and control cells to target cells, comprising the steps of:
a) providing target cells attached to a surface;
b) providing cells of interest and control cells of the cells of interest;
c) contacting the control cells with the target cells to allow the control cells to interact with the target cells;
d) applying a first force to the control cells, wherein the force is in a direction away from the target cells;
e) determine the percentage of control cells contacted in step c) that remain attached to the target cells;
f) perform steps c), d) and e) with the cells of interest instead of the control cells;
g) repeat steps c), d), e) and f) by applying a second force;
h) optionally, perform steps c, d) e) and f) with one or more further forces;
i) select the force to be applied from the first, second and optional further forces, by comparing, with the same force applied, the percentages of cells of interest and control cells as determined in step e) that remained attached.

In one embodiment, the method in accordance with the invention is for selecting a force to be applied in measuring binding of cells of interest and/or control cells to target cells. In another embodiment, the method in accordance with the invention is for selecting a force for sorting control cells and/or cells of interest. In another embodiment, the method in accordance with the invention is for selecting a force for separation of cells of interest. In yet another embodiment, the method in accordance with the invention is for selecting a force for enrichment of cells of interest.

In step a) of the method, target cells are provided, and in step b) cells of interest and control cells of the cells of interest are provided. The target cells in accordance with the invention are defined to be the cells immobilized, *i.e.* attached to a surface. The cells of interest are defined not to be immobilized. It is understood that in accordance with the invention target cells and cells of interest relate to two different cells which are to interact specifically with each other. Which cell is immobilized may not be of importance for determining cellular avidity between the cells. As long as a force can be applied to the cells binding to the immobilized cells, and detachment and/or attachment of cells can be determined, determination of the cell numbers thereof allows one to determine cellular avidity. Hence, one of the target cells and cells of interest expresses a ligand on its surface and the other cell expresses a receptor for that ligand. As control cells, the same cells as the cells of interest or target cells may be utilized but these cells *e.g.* do not express such a ligand or receptor or express a variant thereof which is not functional. If the control cells are control cells of the target cells, these may be immobilized as well. If the control cells are control cells of the cells of interest, these may not be immobilized. Of particular interest and as further described below, in accordance with the invention, cells of interest (or, conversely target cells) can be T-cells or the like, which are to specifically target *e.g.* a cancer cell or other antigen presenting cell presenting a defined antigen on its surface. Hence, accordingly, in methods of the invention the target cells attached to the surface, *i.e.* the immobilized cells, may be cancer cells, and the cells of interest may be T-cells. Conversely, in methods of the invention, one may also choose to have the T-cell immobilized, as a target cell, and have the cancer cell as a cell of interest. The latter may complicate cell sorting of T-cells, but may be advantageous in scenarios where certain cancer cells are difficult to immobilize and/or different cancers cells may be studied.

The target cells are provided attached on a surface. Providing the target cells attached to a surface is well known in the art. For example, a glass or plastic surface may be utilized to attach cells thereto. A surface material may be preferred which allows for detection of attachment to and/or detachment of the target cells, *i.e.* of cells of interest and/or control cells thereof. Such a surface material for instance also may allow for microscopy methods (*e.g.* by being a transparent material). In order to attach cells to the surface, the surface may be pre-treated with a coating such as a polypeptide. Suitable polypeptides include *e.g.* poly-L-lysine or the like. Suitable polypeptides for attachment of target cells that may be contemplated and are known in the art include for example fibronectin, poly-L-lysine, poly-D-lysine, poly-L-ornithine, laminin, collagen, fibronectin, fibrinogen, vitronectin, or osteopontin. In any case, a suitable polypeptide or other suitable coating if needed may be selected such that the target cells that are attached to the surface allow for the target cells to remain attached to the surface when applying a force on the control cells or cells of interest. In other words, when a force is applied on the control cells or cells of interest which allows for these cells to detach from the target cells, the target cells are to substantially remain attached to the surface (see *e.g.* as shown in Figure 4).

In an alternative embodiment, instead of providing control cells of the cells of interest, control cells of the target cells may be provided attached to a surface. In such a scenario, cells of interest are contacted with the target cells and subsequently, cells of interest are contacted with the control cells of the target cells, and in each case the cells of interest that have detached and/or remained attached determined and cellular avidity scores provided for the cells of interest with the control cells of the target cells and with the target cells. These cellular avidity scores can be likewise compared, and from different forces applied, optimal force conditions may be selected.

In another embodiment, instead of providing target cells attached on a surface, a functionalized wall can be provided which provides the control cells and/or the cells of interest with a suitable surface to attach to in the means and methods in accordance with the invention as described herein throughout. A functionalized wall in accordance with the invention presents ligands/receptors in a similar fashion as they are presented on a cell surface, e.g. in a lipid bilayer, or the like. A functionalized wall thus preferably is functionally equivalent to target cells attached to a surface, and mimics target cells attached to a surface.

In step c) the control cells are contacted with the target cells to allow the control cells to interact with the target cells. Hence, in this step, the control cells are introduced on the target cells, *e.g.* by layering the control cells thereon. It is understood that this step of contacting is well controlled. For example, as shown in the example section, the step of contacting may be a defined period of 5 minutes. Of course, the step of contacting may be shorter or longer, for example in the range of about 2 minutes to about 15 minutes.

Subsequently, in step d) a first force is applied to the control cells, wherein the force is in a direction away from the target cells. It is understood that in this step, the force applied may be perpendicular (in the direction of z-axis) to the surface (x,y) to which the target cells are attached, for example when a centrifugal force or acoustic force is applied. The force may also be lateral (x-axis or y-axis), for example when a shear force is applied (see *e.g.* Figure 5). In any case, the force is applied and is controlled such that a defined force is exerted on the cells that interact with the target cells. It is understood that the force that is exerted on the cells attached to the target cells is to be substantially equal, such can be achieved e.g. when using a flat surface as depicted in Figure 5. Other suitable surface shapes may be used (*e.g.* a tube with exerted concentrical force or laminar flow force in the direction of the length of the tube), as long as the force exerted can be substantially equal at a defined surface area, such a surface shape may be contemplated. The force required to move a cell away from the target cell preferably can be detected, *e.g.* via microscopy or other means, to which may be referred to as a cell detachment event. This way, cell detachment events can be monitored and counted.

In step e), the control cells that have detached and/or remain attached in step d) are determined and provide a cellular avidity score for the control cells. By knowing the number of cells that have interacted with the target cells, and knowing the number of cells that remain attached to the target cells, the percentage of control cells that remain attached to the target cells can be determined. The percentage of control cells from the contacting step c) that remain attached to the target cells can be well determined. This can be done by relatively simple means known to the person skilled in the art, *e.g.* by simply providing a defined number of cells to interact with the target cells and, after incubation and applying the force, subsequently collecting detached cells and determining the amount of cells collected, and calculating the percentage that remains bound therefrom. Of course, it may be advantageous and convenient to use microscopy, with which attached cells can be identified and quantified and detachment can be likewise monitored and quantified. As shown in the examples, the z-Movi^{®} device applying an acoustic force is well equipped to do so. Likewise, similar devices may be provided with microscopy or other means to quantify cells, and attachment and/or detachment events, and also utilizing e.g. shear-force or centrifugal forces instead of acoustic force.

With regard to the cellular avidity score, it is understood that this is to express the strength of binding of cells of interest (or control cells thereof) to the target cells. It is understood that where we refer to specific forces applied to cells this may refer to average forces, e.g. such forces may not be fully homogeneous, for example over the contact surface as may be the case with acoustic forces and shear-flow forces (see *e.g.* Nguyen, A., Brandt, M., Muenker, T. M., & Betz, T. (2021). Lab on a Chip, 21(10), 1929-1947) for a description of force inhomogeneities in acoustic force application).

For shear-flow forces, the forces may also not be fully homogeneous, for example since the flow speed near the side walls of a flow channel (*e.g.* with a rectangular cross section) may be lower than in the center of the flow cell (due to the no-slip boundary condition). By choosing a cross section with a high aspect ratio (low and wide) these flow effects may be minimized such that only a few percent of the cells experience a substantially smaller force than the cells in the center of the flow cell. Other methods to mitigate such effects and to specifically select cells that have experienced similar forces may include using flow cell geometries with multiple fluid inlets and/or outlets such that the properties of laminar flow can be used to ensure cells of interest only land in regions of homogeneous force and/or are only selected from regions of homogeneous force. In one example, by using three channel inlets side by side one can use the side channels as sheath flow channels to focus cells of interest inserted into the center channel where the acoustic and/or shear force may be substantially homogeneous. The sheath flow fluid may be the same buffer fluid as is used for the sample cells but then free of sample cells. By increasing the flow speed through the sheath flow channels the cells are more focused and confined to the center of the channel while by reducing the sheath flow speed the cells are allowed to spread out more. Similarly, on the collection side flows in three side-by-side collection channels may be controlled to possibly discard cells flowing close to the channel boundaries and only collecting cells from the center of the channel. By controlling the relative flow speeds of such side channels and the center channel asymmetrically the location of the effective interaction region of the sorting device can be further controlled and cells that have underwent defined forces can be selected and/or detected.

Further means to enable collection of cells from a specific interaction region (and therefore collection of cells that experienced a defined force) include means and methods wherein cells of interest may be provided with a photoactivatable label which may be subsequently activated by illumination with light of a suitable wavelength only in a well-defined interaction region of the device (*e.g.* near the center of a flow channel or in a center region under an (acoustic) force transducer) to photoactivate and/or switch the dye. Subsequently, the cells can be sorted for example using fluorescence activated cell sorting (FACS) and only those cells which are activated are further used according to the methods described herein thereby obtaining the cells on which defined forces have been exerted. This may for example be highly useful for collecting cells that remained attached to the target cells. For example, the target cells and cells bound thereto may be trypsinized thereby obtaining both the target cells and cells that remained bound thereto in a suspension. Alternatively, attached cells can also be simply collected with physical means (*e.g.* scraping) from the area of interest, *i.e.* the surface area with a well-defined nominal force.

For centrifuge forces it is easier to ensure that the force applied is homogeneous across the whole interaction region since such a force does not depend strongly on a location on a surface with respect to a force transducer and/or the wall of a flow channel or sample holder.

Accordingly, in connection to the subject matter disclosed herein, means and methods exist which allows one to exert forces on cells attached to a surface and collect the cells, detached and/or attached cells, on which defined forces have been exerted and determine *i.a.* the amount thereof, *i.e.* number of cells.

It is understood that with regard to the force exerted, the exact forces experienced by cells may also depend on cell size and or other cell properties such as density and compressibility (the force may be a nominal force and not the true force experienced by the control cells or the cells of interest). *E.g.* it may be hard to precisely predict the average cell size, density, compressibility, etc. of the cells and the force may have been calculated based on theory alone or may have been calibrated using test particles with specific (preferably known) properties (see *e.g.* Kamsma, D., Creyghton, R., Sitters, G., Wuite, G. J. L., & Peterman, E. J. G. (2016). Tuning the Music: Acoustic Force Spectroscopy (AFS) 2.0. Methods, 105, 26-33). The force may be such a calculated or calibrated force expressed with units of N (*e.g.* pN) but it may also be expressed without calibration as the input power (Vpp) applied to a piezo element (see Sitters, G., Kamsma, D., Thalhammer, G., Ritsch-Marte, M., Peterman, E. J. G., & Wuite, G. J. L. (2014). Acoustic force spectroscopy. Nature Methods, 12(1), 47-50), as angular velocity squared (*ω*²) in the case of centrifugal force application or as flow speed v and or as shear stress (Pa) in applications using shear forces. As long as the forces exerted by the devices, *e.g.* shear force, acoustic force, or centrifugal forces, but not limited thereto, can be varied and controlled and reproduced in such devices such devices are suitable for the means and methods in accordance with the invention.

As the percentage of cells that remains bound at a certain applied force is indicative of cellular avidity, *i.e.* the larger the percentage of cells that is bound the higher the cellular avidity, it is useful to refer to such a percentage as a cellular avidity score. Of course, one may use a different measure which relates to cellular avidity. One may also refer to the percentage of detached cells instead, wherein conversely a low number is indicative of a relative higher cellular avidity. Instead of percentage, one may also provide the ratio of cells that remain attached divided by the total number of cells that interacted, or provide the ratio for detached cells. One may also, in case of a cellular avidity plot as shown herein determine the area under (or above) the curve. One may also, in case a fixed number of cells is to be provided to a target surface, simply provide the number of cells that have detached and/or remained attached. In any case, as long as a unit is provided that is representative of the number of cells that have detached or cells that have remained attached, relative to the total number of cells that have interacted, such a unit may be contemplated. Such a unit allows for ranking cellular avidities, when comparing *e.g.* different cells of interest. Providing such a unit may be referred to as providing a cellular avidity score.

Hence, instead of determining the percentage of cells that remain attached to the target cells, relative to the cells of the contacting step, in the means and methods any unit may be provided that is representative of the number of cells that have detached or cells that have remained attached, relative to the total number of cells that have interacted, as such a unit can be regarded as a cellular avidity score. A preferred unit of cellular avidity, to which also may be referred to as a cellular avidity score, may be the percentage of cells that remains attached, relative to the cells that have interacted, the latter being set at 100%. While in the current examples a defined end force of 1000 pN is chosen for comparing the cellular avidities, other forces, force ramps or combinations of forces may also be contemplated as forces at which the fractions of bound vs unbound cells are compared.

Step f) comprises the same steps as described above for control cells of cells of interest, but instead utilizes the cells of interest. Hence, in accordance with the method as described above, step f), in carrying out steps c), d) and e), provides for the percentage of cells of interest contacted in accordance with step c) that remain attached to the target cells. This way, for both the control cells and cells of interest, a measure or unit representative of cellular avidity is provided. It is the difference, or ratio, between these two that is important in the current invention.

It is understood that in accordance with the invention, the repeating of the steps c), d) and e) may involve utilizing the same target cells attached to a surface and/or may involve utilizing multiple provided target cells attached to a surface. For example, as shown in the examples, a chip (such as described *i.a.* in Fernández de Larrea, C., et al. (2020). Blood Cancer Discovery, 1(2), 146-154, WO2018083193, and such as available from LUMICKS for the z-Movi^{®} device (see *i.a.* z-Movi^{®}-Brochure_2021.pdf, available from <https://lumicks.com/products/z-Movi-cell-interaction-studies/#brochure>) with target cells attached to its surface may be repeatedly used in the z-Movi^{®} device and cells that have remained bound to the target cells and remain bound to the target layer in a subsequent measurement may *e.g.* be masked in analysis (*e.g.* by the software or by manually identifying these cells and excluding these in a subsequent measurement). Hence, it is understood that repeating steps may be performed with the same target cells attached to a surface or may be performed with further provided target cells attached to a surface. When multiple surfaces with attached target cells are provided, it is understood that of course these are prepared in the same way such that different measurements with each of the multiple surfaces can be compared and are substantially reproducible. Hence, in one embodiment, the method may be repeated with the same target cells attached to a surface, *e.g.* as a monolayer. In step g), steps c), d), e) and f) are repeated by applying a second force. It is understood that the second force is off course different than the first force that is applied. Optionally, in step h) one or more further forces are applied by repeating said steps c), d), e) and f) with one or more further forces.

It is understood that of course multiple measurements may be optionally performed of the different forces applied, *e.g.* as shown in Figures 2 and 3, measurements were carried out in duplicate, and averages and/or standard deviations determined, or the like of the determined percentages and/or units provided in step e).

In step g), once all the measurements have been carried out by applying different forces, a force can be selected by comparing, with the same force applied, the percentages of cells of interest and control cells as determined in step e) that remained attached, or likewise the units representative thereof. The force to be selected involves comparing the percentages/units of cells that remain attached. Preferably, this difference is the largest difference between the cells of interest and control cells. For example, when the measure/unit determined of control cells and cells of interest is the percentage of cells that remain attached after the force has been applied, ideally, the percentage of control cells is preferably below 15% and of the cells of interest preferably above 85%. Hence, preferably a force is selected which allows for obtaining such percentages. It may also be preferred if different forces result in similar percentages, to select the lowest force applied to obtain such similar percentages and/or differences. Selecting the lowest force may allow for more repeated use of target cells attached to a monolayer and/or may be less stressful to cells. Of course, it may depend on the application with which cellular avidity is employed as a means to differentiate between background binding (of control cells) and specific binding (of cells of interest). For example, in a scenario, wherein of a particular cell of interest, *e.g.* a defined CAR-T, cellular avidities are to be subsequently selected which are to be improved and/or reduced, in order to provide for a range of different cellular avidities, it may be desirable to select for a cellular avidity representative of about 50/60% for the cells of interest and about 10% or lower of the control cells thereof. In any case, by varying the forces to be applied and comparing detachment of control cells and cells of interest, a suitable and advantageous force for subsequent means and methods relying on cellular avidity can be selected.

As already described above, instead of determining the percentage of cells that remain attached, other units representative thereof or (inversely) correlating therewith may likewise be used in the means and methods in accordance with the invention. Hence, in one embodiment, a method is provided of selecting a force to be applied, for differentiating between binding of cells of interest and control cells to target cells, comprising the steps of:
a) providing target cells attached to a surface;
b) providing cells of interest and control cells of the cells of interest;
c) contacting the control cells with the target cells to allow the control cells to interact with the target cells;
d) applying a first force to the control cells, wherein the force is in a direction away from the target cells;
e) determine control cells that have detached and/or remain attached in step d) and provide a unit representing the number of cells that have detached or cells that have remained attached, relative to the total number of cells that have interacted in step c);
f) perform steps c), d) and e) with the cells of interest instead of the control cells;
g) repeat steps c) - f) by applying a second force;
h) optionally, perform steps c, d) and e) with one or more further forces;
i) select the force to be applied from the first, second and optional further forces, by comparing, with the same force applied, the units as determined in step e) with the cells of interest and of the control cells.

In another embodiment, a method is provided of selecting a force to be applied, for differentiating between binding of cells of interest and control cells to target cells, comprising the steps of:
a) providing target cells attached to a surface;
b) providing cells of interest and control cells of the cells of interest;
c) contacting the control cells with the target cells to allow the control cells to interact with the target cells;
d) applying a first force to the control cells, wherein the force is in a direction away from the target cells;
e) determine control cells that have detached and/or remain attached in step d) and provide a cellular avidity score for the control cells;
f) perform steps c), d) and e) with the cells of interest instead of the control cells;
g) repeat steps c) - f) by applying a second force;
h) optionally, perform steps c, d) and e) with one or more further forces;
i) select the force to be applied from the first, second and optional further forces, by comparing, with the same force applied, the cellular avidity scores as determined in step e) with the cells of interest and of the control cells.

As said, preferably, the cellular avidity score or unit determined of control cells and/or cells of interest represents the percentage of cells that remain attached after the force has been applied. In one embodiment, a method is provided of selecting a force to be applied, for differentiating between binding of cells of interest and control cells to target cells, comprising the steps of:
a) providing target cells attached to a surface;
b) providing cells of interest and control cells of the cells of interest;
c) contacting the control cells with the target cells to allow the control cells to interact with the target cells;
d) applying a first force to the control cells, wherein the force is in a direction away from the target cells;
e) determine the percentage of control cells contacted in step c) that remain attached to the target cells;
f) perform steps c), d) and e) with the cells of interest instead of the control cells;
g) repeat steps c) - f) by applying a second force;
h) optionally, perform steps c, d) and e) with one or more further forces;
i) select the force to be applied from the first, second and optional further forces, by comparing, with the same force applied, the percentages of cells of interest and control cells as determined in step e) that remained attached.

Preferably, the percentage difference is at least 30%, or more. As said, preferably, a force may be selected with the largest difference between the percentages. Preferably, the percentage of control cells remaining attached is respectively 15% or below, and of cells of interest this preferably is in the range of 85% or higher. More preferably, in a scenario wherein e.g. a force is to be selected for sorting purposes and the like, it may be desirable to have no control cells remaining attached, or at least a low percentage, such as 5% or less, 4% or less, or 3% or less. With any other unit or cellular avidity score determined, optimal differences can likewise be selected.

The method as described above involves providing cells of interest. It is also possible to only provide control cells, and accordingly, not include cells of interests in the steps of the method. Hence, in another embodiment, the means and methods as described herein for selecting a force to be applied may comprise not providing cells of interest in the method nor determining an avidity score thereof. For example, one may solely determine the force with which a low percentage of control cells remain attached to the target cells. This way, by selecting such a force, under these conditions it may be possible to select for cells of interest subsequently, *i.e.* cells of interest with desirable or predefined desirable cellular affinities relative to the control cells.

Hence, in another embodiment, a method is provided of selecting a force to be applied, for differentiating between binding of cells of interest and control cells to target cells, comprising the steps of:
a) providing target cells attached to a surface;
b) providing control cells of cells of interest;
c) contacting the control cells with the target cells to allow the control cells to interact with the target cells;
d) applying a first force to the control cells, wherein the force is in a direction away from the target cells;
e) determine control cells that remain attached to the target cells and/or control cells that detached from the target cells;
f) repeat steps c) - e) by applying a second force;
g) optionally, perform steps c, d) and e) with one or more further forces;
i) select the force to be applied from the first, second and optional further forces, by comparing the determined control cells that remain attached to the target cells and/or control cells that detached from the target cells. Of course, this may also and preferably determining a cellular avidity score.

As already described above, the step of determining the control cells that remain attached may also comprise alternative measurements, *e.g.* determining cells that detach from the target cells in step d) above. Whichever way, a cellular avidity score or a unit is determined representing a binding strength of the target cell-cell of interest bond, such as a cellular avidity score representing the number of cells that have detached or cells that have remained attached, relative to the total number of cells that have interacted in step c). Preferably, the force to be selected is a force with which the percentage of control cells that remain attach is lower than 50%, preferably lower than 35%, more preferably lower than 15%. By selecting such a low force that provides for such low background binding of control cells of cells of interest, advantageously in subsequent assays, cells of interest can be selected with a relative higher avidity as compared with the control cells and the low background binding allows to differentiate between different relative cellular avidities of potential different candidate cells of interest.

Hence, the means and methods in accordance with the invention as described herein, do not necessarily require a priori the provision of cells of interest in order to select a suitable force. Hence, it is understood that the means and methods as describe herein can be performed without providing cells of interest and solely apply the different forces on the control cells.

Any suitable force application method may be contemplated in accordance with the invention. Increasing the force can be well controlled with acceleration based methods of applying force such as centrifugation, with shear flow and with acoustic force, which are all suitable means to be used in the methods in accordance with the invention but any other means of controllably causing a force on the cells attached to the target cells thereby forcing them away from the target cells or the functionalized wall surface, may be contemplated. In a further embodiment, the applied force in the means and methods of the invention is a force ramp, preferably a linear force ramp. It is understood that the different forces that are to be applied can be constant forces applied for a defined period. The forces applied may be in various forms as a function of time. Preferably however, the applied force is an increasing force, that is, after the incubation step, an increasing force is applied for a defined period until a defined end force is reached. For example, as shown in the example section, in about 150 seconds, a defined end force is reached of 1000 pN or 3000 pN. Increasing the force is preferably done in a linear force ramp, but other ways to increase the force over time may also be used (*e.g.* exponential loading where the force is doubled over a certain time period and keeps doubling until a defined end force is reached).

The different forces (of the first, second and optional further forces) that are applied thus may include different force loading rates and/or a defined (end) force. A defined end force in the case of different force loading rates can be the same or can be differently selected end forces. Hence, in case of varying force loading rates, the time period of the applied force loading rate can be varied or can be selected to be the same for each force loading rate. Preferably, the force loading rate can be a linear force loading rate, which can be expressed as *e.g.* pN/s. It is understood that when reference is made to the forces applied this relates to the forces that are applied relative to the cells of interest or control cells of the cells of interest (see also below).

Hence, in a further embodiment, for the different forces applied as a first, second, and optional further force, wherein the forces are applied as a linear force ramp, includes providing for each linear force ramp a force loading rate and an end force.

In any case, when applying different force ramps, with different force loading rates, with different and/or the same end forces, the results obtained therewith can be compared and subsequently a force loading rate and time applied may be selected therefrom. Hence, a force loading rate and/or time period for application of the force loading rate can be selected in the step g). This may be the same force loading rate and time applied in the method. Alternately, a shorter time may be selected for applying the force loading rate when for example cellular avidity curves shows a flattening of the curve and additional time and increased force may not be much informative. For example, as shown in Figure 2, two different force loading rates were tested, 20 pN/s for 150s, and 6,7 pN/s for 150s, with defined end forces of 3000 pN and 1000 pN respectively. As can be observed in Figure 2, the biggest difference between background binding (UNT) and cells of interest (CAR) is obtained with a 20 pN/s for 150 seconds. However, when we look closely at Figure 2, from about 100 seconds, the additional 50 seconds in this scenario do not contribute much, *i.e.* cells of interest remain bound and no substantial amount of cells are detached further. Hence, when comparing the applied forces a shown in Figure 2, it would be advantageous to select a force loading rate of 20 pN/s and apply this for 100 s, arriving at an end force of 2000 pN. This way, it is not necessary to apply a further force up to 3000 pN. Not applying a further force and increased force can be advantageous as it may allow for more measurements to be performed. It should be noted that although it has been demonstrated that cells remain viable under these types of force applications (see *e.g.* Figure 4) it may still be advantageous to limit the force applied to both the surface bound target cells as well as the control cells and/or cells of interest. It may be advantageous to limit the forces applied and the duration of the forces applied as much as possible as it may reduce cell perturbation. Hence, in one embodiment, the method in accordance with the invention, in the selection step of a force, from the selected force ramp the force rate and the end force is selected. In another embodiment, advantageously, in the selection step of a force, from the selected force ramp the force rate is selected and a lower end force, which lower end force being lower than the end force of the selected force ramp and showing no substantial further cell detached from the target cell attached to the surface. In another embodiment, in the selection step of a force, from the selected force ramp the force rate is selected and a lower end force. As said, the applied force, be it a force ramp, direct force, or a linear force ramp, may be an acoustic force, a shear flow or a centrifugal force. It may be advantageous to apply shear flow and/or centrifugal flow in scenarios wherein large number of cells are to be tested. As shown in the examples, the steps c), d) and e) may be performed with control cells and with cells of interest in separate measurements. Of course, the steps c), d) and e) may also be performed by combining the control cells and cells of interest in a combined measurement, for example when it is possible to distinguish between cells of interest and control cells, *e.g.* carrying different (fluorescent) labels or the like. Hence, in another embodiment, in the method in accordance with the invention, the steps c), d) and e) with the control cells and step f) with the cells of interest are performed with the control cells and cells of interest combined. In another embodiment, in a method in accordance with the invention, the contacting step c) of the control cells and of the cells of interest, and subsequent steps d) and e) are performed separately or these steps are performed with the control cells and cells of interest combined.

In it is understood that *e.g.* when further subsequent measurements are performed using the same target cells attached to a surface that at the end of each force application, the cells are to be removed such that these cells can no longer interact with the target cells, which may disturb further measurements. Of course, one may also use after a measurement a new surface with attached target cells which has been in the same manner. Any of the cells that remained attached to the target cells may be masked in a subsequent analysis (*i.e.* not taken into account in the analysis). Hence, it is understood that repeated use of surface with attached target cells may be preferred in cellular avidity measurements or the like, and perhaps less useful for sorting, and the latter may require that all cells that detached and that remained attached are to be removed.

In another embodiment, the target cells are attached to a glass surface, preferably a glass surface in a chip (*i.a.* as described in WO 2018/083193, and such as available from LUMICKS for the z-Movi^{®} device (see *i.a.* z-Movi^{®}-Brochure_2021.pdf, available from <https://lumicks.com/products/z-Movi^{®}-cell-interaction-studies/#brochure>). The target cells that are attached to the surface, preferably are attached as a monolayer. The monolayer preferably is at high confluency. The subsequent cells of interest (and control cells thereof) that are to interact with the target cells are preferably provided in a relatively low cell density as compared with the target cells, such that substantially all cells of interest (and control cells thereof) can interact with a target cells (there are more target cells per cell of interest). Such provides for advantageous controllable conditions when applying the force on the control cells or cells of interest.

With regard to the target cells and cells of interest and control cells thereof, it is understood that this may involve cells that are to have a specific interaction, *i.e.* one cell carrying a receptor and the other cell having a ligand for the receptor. The term ligand and receptor in this sense and in accordance with the invention referring to defining their inter-relationship. The term receptor may not be construed to be limiting in any way and is understood to mean a protein presented at the cell surface which can (specifically) interact with another protein (ligand) presented at a another cell. The terms ligand and receptor are used to indicate a complementarity which is important for specific recognition between cells without restrictions on the complementary molecules that can be contemplated. For example, one cell may have a T cell receptor or a CAR-T, and the other cell may present an antigen for the T-cell receptor, e.g. presenting an antigen via MHC. Such interactions are of interest to study and to modify. Hence, such cells of interest, and control cells thereof not carrying e.g. the CAR-T, and target cells are in particular of use in the means and methods of the invention, as exemplified in the example section herein. In a preferred embodiment immune effector cells are provided as cells of interest, and target cells expressing an antigen which the cell of interest is to specifically target. For example, effector cells may be selected from T cells, NK cells, dendritic cells, macrophages, or monocytes. Such cells may be genetically modified, *e.g.* provided with *e.g.* a CAR. As said, target cells may be cancer cells, or other cells expressing an antigen, e.g. viral antigens or the like.

Once a force is selected in the last step of the method, this can be subsequently used in advantageous methods that rely on cellular avidity and for which it is important to be able differentiate between different cellular avidities. Cellular avidity may be defined as the percentage of cells that remain attached to the target cells. Of course, any other unit, such as described above, may be equally selected as a measure of cellular avidity. As in this case, the forces applied provide for further information related to the cellular avidity.

Hence, in further embodiments in accordance with the invention, a method for determining cellular avidity is provided comprising:
i) providing a selected force in accordance with the invention and as described above;
ii) providing target cells attached to a surface;
iii) providing cells of interest;
iv) incubating the cells of interest with the target cells attached to the surface;
v) determining the cellular avidity score of the cells of interest to the target cells by applying the selected force on the cells of interest and determine cells of interest detaching from the target cells and/or cells of interest remaining attached to the target cells.

Such further methods relying on the selected force in accordance with the invention may be carried out with the target cells attached to the surface used in the method of selecting the force, but may of course also be performed with newly provided target cells attached to a surface. It is understood that such newly provided target cells attached to a surface are of course prepared in the same way as used in the methods of selecting the force. Of course, in these further methods, control cells of the cells of interest can be provided as well, and steps iv) and v) can be performed therewith.

A parameter of interest in addition to cellular avidity may be the ratio between the cellular avidity of the cells of interest and the control cells. For example, the higher the ratio, it may be indicative of the cell of interest and target cell interaction being more selective, and may allow e.g. to compare different cell of interest and/or different target cell interactions.

Advantageously, of a variety of cells of interest the cellular avidity may be determined. This is for example of interest when it is desirable to provide for a candidate CAR, a variety of cellular avidities. By providing a variety of CARs, candidates can be selected and subsequently assessed with regard to functionality, *e.g.* by *in vivo* experiments. As cellular avidity is an important parameter for function, this way highly efficiently, suitable candidates can be selected.

Of course, the selected force is not only useful for determining cellular avidity, but can also be utilized in other methods for other purposes. For example, further methods can include methods for identifying a candidate agent capable of modulating the cellular avidity between a cell of interest and a target cell, wherein in the steps i) to iv) of the method as defined above, agent(s) are provided. This way, when it is for instance of importance or interest to block or enhance a particular interaction, agent(s) capable of doing so can be identified. Furthermore, a selected force can also be used in methods for screening of different cells of interest, by performing the method as defined, optionally in the presence of agent(s) as defined above, and comparing determined cellular avidities for the different cells of interest. This way, for example, in case agents are present that are known to modulate a desired interaction between a cell of interest and a target cell, receptor/ligand interactions may be selected that are not affected by such agents, or, conversely, are aided by such agents. Of course, once such a force has been selected, and/or agent, and shown to provide for selectivity with regard to binding of cells of interest to the target cells, as opposed to control cells, such can also be used for selecting and/or sorting cells of interest. Hence, in another embodiment, a method is provided for selecting and/or sorting cells of interest, comprising the steps i) to iv) of the method as defined above, and optionally in the presence of agent(s) as defined above, comprising the further step of applying the selected force on the cells of interest and subsequently selecting and/or sorting cells of interest that have detached and/or that remain attached to the target cells.

Cells of interest which can be selected and/or sorted and thus be obtained accordingly with means and methods in accordance with the invention may subsequently in a final step be admixed with a pharmaceutically acceptable buffer or otherwise pharmaceutical acceptably formulated.

In another embodiment, a cell engager may be provided. Cell engagers include antibodies, or the like, which are capable of binding to a target cell and cells of interest, *e.g.* an effector cell. Such antibodies may include single chain antibodies comprising two binding domains such as scFv domain, and include BiTEs (*i.e.* bispecific T cell engagers) or the like. A conventional antibody design includes heavy and light chains, with one half of the antibody (one heavy chain and one light chain) engaging with a target cell, and the other half of the antibody (another heavy chain and another light chain) engaging with an effector cell, wherein preferably, the Fc domain is made inert. In any case, suitable cell engagers are widely known in the art and the current invention allows to study and/or determine cellular avidity, *e.g.* synapse formation, of induced by a cell engager between a target cell and an effector cell. Hence, accordingly, in the means and methods in accordance with the invention, a cell engager may be provided in addition, and *e.g.* the cellular avidity score is determined, induced by said cell engager between a cells of interest (*e.g.* an effector cell) and a target cell, said cell engager having a binding region capable of binding the cells of interest (*e.g.* effector cells) and a binding region capable of binding the target cell. It is understood that the incubation step in the methods of the invention can thus be performed in the presence of the cell engager to allow the cells to interact, *i.e.* effector cells and target cells, and form a bond, *e.g.* a synapse, via the cell engager.

Accordingly, in one embodiment, in methods in accordance with the invention, a cell engager is provided capable of binding an effector cell and the target cell and inducing synapse formation, and the cell engager is included in the incubation step, *i.e.* in the step of contacting target cells with cells of interest or with controls thereof. Furthermore, such methods are highly useful for screening cell engagers, *e.g.* by identifying cell engagers that are particular capable of inducing a synapse.

It is understood that a synapse is a specialized structure that forms when the plasma membranes of two cells come into close proximity to transmit signals. Synapses can form between cells of interest and target cells, when *e.g.* the cells of interest are effector cells. Cells of the immune system form synapses that are essential for cell activation and function. Lymphocytes such as T cells, B cells and natural killer (NK) cells form synapses that can be referred to as immunological synapses. Such a synapse typically forms between effector cells and target cells, *e.g.* cells presenting an antigen. A non-limiting example is *e.g.* a T cell or a CAR-T cell and a cancer cell. The formation of synapses between *e.g.* an effector cell and a target cell, for example an APC (antigen presenting cell), is a hall-mark event and signals the presence of specific interactions (*i.e.* the specific interaction between, for example, a TCR or CAR and an antigen recognized thereby) between the effector cell and the target cell that are involved in the formation of such immunological synapses.

In the case of a T-cell, a synapse can be formed between the lymphocyte and antigen-presenting cells (APCs) during the recognition of the peptide antigen-major histocompatibility complex (pMHC) ligand by the T-cell antigen receptor (TCR). The TCR and pMHC are both membrane-bound so the TCR will only be triggered by its ligand at the interface between T-cells and APCs. A synapse can be observed at the T-cell - APC interface as concentric rings by confocal microscopy, often referred to as "bull's eye" (Huppa, J. B., & Davis, M. M. (2003). T-cell-antigen recognition and the immunological synapse. Nature Reviews Immunology, 3(12), 973-983). These rings were named the central, peripheral, and distal supramolecular activation cluster (*i.e.* respectively cSMAC, pSMAC and dSMAC). The TCR has been reported to be present in the cSMAC, whereas other lymphocyte specific proteins such as lymphocyte function-associated antigen-1 (LFA-1), are integrated into the pSMAC ring that surrounds the TCR. The formation of this ringed structure ("bull's eye") is however not universal and other formations such as "multifocal immunological synapses" between T-cells and dendritic cells, or the like, have been described.

The immunological synapse can be considered to be any structure formed at the interface resulting from a functional and specific effector-target cell interaction, such as for example T-cell-APC contacts. Markers associated with effector cells and synapse formation include one or more of CD43, CD44, CD45, LFA-1, Talin, F-actin, ZAP70, CD2, CD4, CD8, CD3, CD28, PD-1, ICOS, and TCR. Markers of target cells include one or more of ICAM-1 (associates with LFA-1), CD48/58 (interacting with CD2) CD80/CD86 (interacting with CTLA-4 and CD28), PDL1/PDL2 (associating with PD-1), and MHC presenting the antigen (that specifically interacts with the TCR). These markers, and concentrations thereof, may be detected *e.g.* with fluorescent labels at the interface between effector cell and target cell, or intracellularly in close proximity to the synaptic interface.

For example, markers of effector cells that have been associated with dSMAC are CD43, CD44, CD45. The effector cell markers LFA-1, Talin, F-actin, CD2, CD4 and CD8 have been associated with pSMAC. The markers CD3, CD28, PD-1, ICOS, and TCR of effector cells have been associated with cSMAC. Markers that have been associated with a synapse on a target cell are ICAM-1 (associates with LFA-1), CD48/58 (interacting with CD2) CD80/CD86 (interacting with CTLA-4 and CD28), and PDL1/PDL2 (associating with PD-1), and of course an MHC presenting the antigen (that specifically interacts with the TCR). These markers have been shown to be associated with synapses in a TCR-MHC interaction. Likewise, cell engagers (such as a bispecific antibody that e.g. binds CD3 on an effector cell with one arm and with the other arm an antigen on a target cell) which engage an effector cell with a target cell, can trigger the formation of a similar synapse structure as observed with a classic TCR-MHC interaction.

With regard to CARs of e.g. CAR T cells, these are chimeric antigen receptors that are to mimic a TCR or the like. CARs are engineered. The first generation of CAR were provided with an antigen recognition part often an antibody derived region (e.g. a scFv) fused to a transmembrane region and intracellular region of *e.g.* a CD3 ζ-chain. Later generations combined intracellular signalling domains from various costimulatory protein receptors (e.g., CD28, 41BB, ICOS) incorporated in the cytoplasmic tail of the CAR to enhance signalling further. Further generations also incorporated in their design an inducible release of transgenic immune modifiers, such as IL-12, to shape the tumor environment by augmenting *e.g.* T-cell activation, attracting and activating innate immunity. As CARs often have antibody variable regions incorporated, these can target e.g. receptors themselves that are presented at the surface of a cell (*e.g.* Her2, PD-1 etc.), or can also target antigens presented by MHC, derived *e.g.* from proteins intracellular processed by the ubiquitin-proteasome system. Such peptides presented by MHC include proteins that are processed internally and presented by MHC, which can be derived from receptors, secreted proteins, intracellular proteins or internalized proteins. Synapses formed between CARs and target cells may not provide a classical bull's-eye like structure with a well-characterized SMAC domain, but may result in less organized pattern. Multiple CAR microclusters form and signalling molecules, which are dispersed in the center of the synapse interface.

Synapse formation is a spatiotemporal process that starts *e.g.* by TCR binding to MHC or binding of an antigen with CAR or cell engagement, and subsequent phosphorylation of the cytosolic tails of CD3 resulting in a triggered state. This sets of a cascade of processes that result in an activated T cell state, which also depends on the effector cell type and its phenotypic state. Key processes include: calcium signalling, internal cell structure and/or cytoskeleton changes of effector cells, involving F-Actin, Talin, and changes in microtubules, centrosomes, lytic granules, nucleus position, and mitochondrial location. Transactivation of adhesion molecules, cytokine and marker expression. IFNγ, granzyme and perforin may be released by effector cells to thereby induce *i.a.* target cell killing. Also, in addition, in target cells apoptotic markers can be found, including ICAM-1 clustering, phosphatidyl translocation, mitochondrial depolarization, caspase-3 activation and DNA fragmentation. Hence, various stages of specific effector cell and target cell interaction and immune activation can be detected.

In any case, synapse formation, or a synapse can be determined by staining, *i.e.* with fluorescent labels, ligands, antibodies, or probes, or the like, which may be used on live cells or on fixed cells targeting the mechanisms involved in T-cell activation and/or synapse formation. Sequencing based methods may also be used to identify activated T-cells and thereby associate mRNA levels with the formation of stable synapses. T-cell activation leads to changes in mRNA stability and expression. *E.g.* increases in expression of cytokine or secretory transcripts (IL2, IFNγ, granzyme, perforin) and proliferation pathways and either bulk or single-cell RNA sequencing may be used to detect these changes and correlate these to the number or synapse formed.

In any case, in the cells obtained, which can be either in the form of target cells bound to effector cells or separated cells, *e.g.* with trypsin, the marker associated with synapse formation can be determined. Thus, in a further embodiment, the marker associated with synapse formation is determined in either the target cell or the effector cell. In another embodiment, in the methods in accordance with the invention one or more markers associated with synapse formation are determined and the one or more markers are determined in the effector cells and/or in the target cells. When cells are labelled, either in a singlet state or doublet state, cells may be highly advantageously be sorted and collected and subsequently analysed.

In one embodiment, a method in accordance with the invention is provided, wherein the marker associated with synapse formation is selected from the group consisting of calcium signalling signatures; spatial clustering of synapse localized molecules such as LFA-1, CD28, CD3, Agrin; changes to internal cell structure and/or cytoskeleton such as F-Actin, Talin, microtubules, centrosome, lytic granules, nucleus position, mitochondrial relocation; changes in effector cell motility; changes in external cell morphology and/or cell shape; and apoptosis of target cells. Synapse formation includes the initiation of a synapse up to and including the establishment of a synapse in which, as described above but not necessarily limited thereto, markers associated with synapse formation are associated.

In a further embodiment, the marker for synapse formation is calcium signalling, which can be detected with fluorescent calcium indicators, such as Fura2 AM (available from Invitrogen, item nr. F1221), which marker is suitable for detection in effector cells and in live cells. Other suitable dyes to detect calcium signalling can be selected from the group of Fura Red AM, Indo-1, pentapotassium, Fluo-3, fluo-4, Calcium Green-1, Rhod-2 and X-Rhod-1, Oregon Green 488 BAPTA. Hence, in one embodiment, the marker for synapse formation is calcium signalling which is detected with an indicator selected from the group consisting of Fura2 AM, Fura Red AM, Indo-1, pentapotassium, Fluo-3, fluo-4, Calcium Green-1, Rhod-2 and X-Rhod-1, and Oregon Green 488 BAPTA. With these markers calcium signalling can be detected which is a hallmark of synapse formation.

Furthermore, membrane potential dyes may also be used a calcium signalling indicators, such as the Invitrogen FluoVolt^{™} Membrane Potential Kit (Catalog number: F10488). Depolarization of the synapse forming cells by using a slow-response potential-sensitive probe such as Invitrogen DiSBAC2(3) (Bis-(1,3-Diethylthiobarbituric Acid) Trimethine Oxonol), Catalog number: B413. Hence, in another embodiment the marker for synapse formation is detected utilizing membrane potential dyes.

In another embodiment, the marker for synapse formation is cytoskeleton rearrangement, which can be detected in effector cells with live or fixed cells, using cell staining, *e.g.* F-actin can be detected with Phalloidin conjugates or CellMask^{™} (Invitrogen, item nr A57243). Other usable stains can include SiR-Actin, CellLight^{™} Talin-GFP, BacMam 2.0, or Tubulin Tracker Deep Red. Hence, in one embodiment, the marker for synapse formation is cytoskeleton rearrangement, which is detected with a stain selected from the group consisting of Phalloidin conjugates, CellMask^{™}, SiR-Actin, Cell Light^{™} Talin-GFP, BacMam 2.0, or Tubulin Tracker Deep Red. With these markers, cytoskeleton rearrangement can be detected.

In another embodiment, the marker for synapse formation involves monitoring effector cell motility. Detection of effector cell motility can be performed by video/timelapse monitoring of effector cells that remain in contact with the target cells after the force has been exerted. Detection of synapse formation includes detecting effector cell immobility, *i.e.* upon synapse formation effector cells will stop moving and remain into contact with the target cell with which it forms a synapse. Cell motility can be detected by membrane staining of effector cells and imaging, or using brightfield, darkfield or phase contrast microscopy.

It is understood that for some of the methods for detecting a marker for synapse formation which use *e.g.* microscopy and monitoring of motility, or short-lived signals, may be combined with having cells provided with *e.g.* photoactivatable label, to, upon detection of the marker, activate such a label in the cell, such that in subsequent steps, *e.g.* sorting, FACS analysis or the like, cell for which the marker associated with synapse formation was detected can easily be tracked.

As described above, in accordance with the invention, markers associated with synapse formation can be determined, *e.g.* via utilizing labels or the like. However, it may be highly advantageous to sequence obtained cells and identify synapse formation by sequencing. It is understood that sequencing comprises nucleotide sequencing, *e.g.* sequencing of DNA and/or RNA as expressed in cells. Means and methods are widely known in the art to sequence DNA and/or RNA as expressed in the cell. Hence in another embodiment, in the methods in accordance with the invention the markers are determined with sequencing. This is in particular highly useful for such markers which are up- or downregulated in target cells and/or effector cells in forming a synapse or having a synapse. Suitable markers for T-cell activation and synapse formation are transcripts linked to interferon expression, proliferation, and cytokine expression and include: Interferon pathway upregulation: CD4, IFIT3, IFIT2, STAT1, MX1, IRF7, ISG15, IFITM3, OAS2, JAK2, SOCS1, TRIM21; proliferation: LIF, IL2, CENPV, NME1, FABP5, ORC6, G0S2, GCK; cytokine expression: CCL3, IFNG, CCL4, XCL1, XCL2, CSF2, IL10, HOPX, TIM3, LAG3, PRF1, TNFRSF9, NKG7, IL26. Sequencing may also be used to identify non-synapse forming cells by detecting molecular signatures linked to resting cell states: FOXP3, CTLA4, MTNFRSF4, IRF4, BATF, TNFRSF18, TOX2, PRDMI, LEF1, ATM, SELL, KLF2, ITGA6, IL7R, CD52, S100A4, TGFB3, AQP3, NLRP3, KLF2, ITGB7.

It may be also advantageous to determine the molecular state of the target cells as this can give an indication of the cell killing potency of the effector cells. In the case of paired analysis were effector-target-cell doublets are recovered this enables direct linking of effector cell phenotype with their killing capabilities. Next to staining methods for apoptosis commonly used in the field, sequencing or qPCR can be used to detect transcripts linked to apoptosis or cell survival in the target cell, markers include: Bcl-2 family (BCL-xL) caspases 3, caspases 7, cleaved PARP, bax, bad, bak, bid, puma noxa, bcl-2, bcl-xl, mcl-1, p53, and cytochrome c, Smac/ Diablo, survivn, Mcl-1, RNA Y1.

As it is understood that changes in gene expression may take some time and are not necessarily immediately detectable, one may allow after the step of exerting the force, the cells to remain bound to the target cells for some time and have these remain attached to the surface as well. Alternatively, one may also separate *e.g.* doublets that remained after exerting the force and subsequently *e.g.* sort doublets in single wells, and allow these to remain for some time. A suitable time to allow for expression of markers associated with synapse formation may be from 1 hour to 24 hour.

This is highly advantageous as sequencing methods allow for single cell sequencing, which also allows for determining *e.g.* the sequences of receptors expressed by the cell as well. Hence, in a further embodiment, different receptors are determined and identified via sequencing. In yet another embodiment, sequencing comprises single cell sequencing. In still another embodiment, sequencing comprises sequencing the expressed genome. Sequencing the expressed genome is highly useful as it allows to determine up- and downregulated gene expression. Nevertheless, sequencing genomic DNA may be useful as it may also provide useful information *e.g.* epigenetic markers associated with *in vivo* potency and durable responses.

With regard to the methods for determining cellular avidity, *e.g.* in screening, selecting, and/or sorting as described herein, which may include determining *e.g.* a cellular avidity score, as described herein, in accordance with the invention, it is understood that this can take into account the number of cells that have remained bound to each other after applying the force on the cells of interest. In such methods, wherein the cells of interest are effector cells, advantageously, this may also take into account the number of cells that remained bound to each other having one or more markers associated with synapse formation. For example a cellular avidity score, or determining cellular avidity, which takes into account target cells and effector cells having a marker associated synapse formation may provide for a more accurate cellular avidity score which is more reflective of the function of *e.g.* effector cells, *i.e.* forming synapses with target cells. Hence, such a cellular avidity score may also be referred to as a functional cellular avidity score or a synaptic cellular avidity score, and it is understood that where herein cellular avidities or cellular avidity scores are determined in accordance with the invention, this may also include cellular avidity scores taking into account synapse markers. Hence, in further embodiments, synapse markers are used in the methods of the invention such as in screening, selecting, sorting and/or screening of cells of interest, *e.g.* of effector cells. It is also understood that with regard to selecting the force to be applied for differentiating between binding of cells of interest and control cells to target cells, in case of cells of interest are capable of forming a synapse with target cell, *e.g.* when cells of interest are effector cells, synapse markers may also be used in determining cellular avidity scores and selecting the force to be applied. It may also be contemplated to not use control cells at all, as a high percentage of cells of interest bound to target cells may be indicative of low background binding. Thus, in one embodiment, in the methods in accordance with the invention, wherein the cells of interest are effector cells of the effector cells bound with the target cells after the step of applying the force away the presence of a marker associated with synapse formation is determined. In a further embodiment, in the methods of the invention after the step of applying the force away from the target cells, the cellular avidity scores are determined based on the number of effector cells associated with the presence of a marker associated with synapse formation.

It was observed that when cells that remained bound and attached after a cellular avidity measurement were resuspended utilizing *i.a.* repeated pipetting and thus exerting a significant force, cells attached to the surface were detached and moreover, a portion of the cells that were bound to the attached cells became unbound. Hence, this implied that with such a process step a differential force can be applied on bound cells that can exceed the maximum force that is applied away from cells attached to a surface. This way, further cell-cell bonds that may be aspecific cell-cell bonds may thus be broken therewith providing substantially specific cell-cell bonds that remain, e.g. effector cells bound with target cells (and optionally a cell engager) that formed a synapse can be retained. Hence, in a further embodiment, after the force has been applied away from the attached cells, subsequently, the cells are resuspended and a differential force is applied such that substantially effector cells that are bound to each other via a synapse remain bound to each other and substantially cells that have formed aspecific bonds are unbound, *i.e.* have their cell-cell bonds broken. It is also understood that instead of applying the force away, the differential force may be applied instead, and that by applying a differential force, it may no longer be required to have cells attached to a surface, *i.e.* immobilized. Furthermore, it is also understood that after the differential force has been applied, cells are highly preferably separated and/or sorted in order to avoid further interaction between cells to avoid establishing additional cell-cell bonds. Hence, highly preferably, cells are collected and sorted and/or analysed after the differential force has been applied.

As is clear from the above, the type of force that is to be applied in accordance with the invention is a force capable of breaking cell-cell bonds, *i.e.* the force exerted causes cells bound to each other move away from each other to such an extent that a cell-cell bond may break or rupture. A differential force means that the force on one cell differs from the force on the other cell with regard to direction of the force and/or the magnitude of the force, resulting in a net force allowing to break cell-cell bonds if the differential force exceeds the binding force.

For example, when a target cell bound to an effector cell, a doublet, is forced through a nozzle, the closer to the throat of the nozzle the faster the flow. This means that the first cell to enter the nozzle is subjected to a stronger acceleration than the cell lagging behind and the cells experience a differential force resulting in a net force which can result in cell-cell bond rupture, provided the force is large enough (see *e.g.* Figure 6, in particular 6d). A differential force that can be applied includes a shear force, *e.g.* such as can be applied utilizing repeated pipetting (repeated upwards and downwards flow of the sample) or flow through a nozzle.

Other means and methods are known in the art with which shear forces can be applied to cells, e.g. flowing a cell suspension at a constant speed and bombarding these cells to a flat surface at a defined angle. Furthermore, forcing a cell suspension through a needle with a defined internal diameter and a defined force may provide for a well controllable shear force as well. The cell suspension may be subjected to several rounds of such process steps to ensure substantially all cell-cell bonds experience the maximum force that may be achieved with the process step. Such a process step allows for automation, enabling control and repeatability of the process, therewith controlling shear forces exerted. Suitable devices for breaking apart cell-cell bonds which are not synapses are known in the art (*e.g.* Zahniser et al., J Histochem Cytochem. 1979, 27 (1), 635-641). Also, by properly tuning the forces in a flow-cytometer normally used to measure cell deformations, such as *e.g.* described in Otto, et al. Nat. Methods 12, 199-202 (2015) suitable forces can be applied. Tuning can be achieved *e.g.* by changing the nozzle size or geometry and/or the flow speeds used. Other suitable devices known in the art may include for a vortex mixer, with which shear forces may be suitably applied as well. Accordingly, in one embodiment, the force applied involves a shear force.

In another embodiment, the force applied is an ultrasonic force. It is understood, as outline above, that such ultrasonic forces are not forces such as applied *e.g.* in a device as available from Lumicks, wherein the force is away from attached cells (*e.g.* such as in the LUMICKS z-Movi^{®} Cell Avidity Analyzer, *e.g.* as used by Larson et al., Nature 604)7906):1-8, April 13, 2022). It is also understood that the ultrasonic force is selected such that cells are not lysed. Hence, appropriate ultrasonic forces can be applied to cells such that cell-cell bonds can be ruptured, which more preferably includes breaking aspecific cell-cell bonds and less preferably breaks specific cell-cell bonds in which an immune synapse is formed. Examples of using ultrasonic forces to break (aspecific) cell-cell bonds, are known in the art (*e.g.* as described in Buddy et al., Biomaterials Science: An Introduction to Materials in Medicine, 3rd edition, 2013, Chapter II.2.8, page 576; and Moore et al., Experimental Cell Research, Volume 65, Issue 1, 1971, Pages 228-232).

In any case, suitable applied forces which are known in the art include *e.g.* a force in the range of 50 pN - 10 nN, which said force is a net force exerted on one cell relative to the other cell, of two cells bound to each other. Which means the force is exerted on the cell-cell bond. In another embodiment, the force exerted on one of the two cells relative to the other cell is at least 50 pN, or at least 100 pN, or at least 200 pN. In another embodiment, the force exerted is at most 10 nN, at most 5 nN, at most 3 nM, at most 2 nM, or at most 1 nN. In yet another embodiment, the force is selected from the range of 1 pN - 10 nN, from 100 pN - 10 nN, from 500 pN - 10 nN, from 1 nN - 10 nN. In still a further embodiment, the force is selected from the range of 500 pN - 5 nN, from 500 pN - 4 pN, from 500 pN - 3 pN. For example, a suitable amount of force that can be exerted between cells (*e.g.* such as in the z-Movi^{®} device) can be selected to be in the range of 200 pN - 3000 pN. Of course, these force ranges are known to be useful with cells attached to a surface, and the maximum force that may be selected may exceed 3000 pN as it is not required to have the cells attached to a surface in accordance with the invention.

Accordingly, it is understood that in these embodiments, the differential force to be applied does not require either of the target cells or cells of interest, *e.g.* effector cells, to be attached, and the differential force is a force selected from the range of 50 pN - 10 nN. In another embodiment, in methods in accordance with the invention wherein the force that is applied is a differential force, neither the target cells nor the cells of interest, *e.g.* effector cells, require to be attached to a surface, and the differential force is applied in the range of 50 pN - 10 nN, thereby providing cells substantially comprised of target cells, cells of interest, and cells of interest bound to target cells. It is understood that by selecting an appropriate force, aspecific cell-cell bonds may be selectively broken, while retaining specific cell-cell bonds between cells of interest and target cells. For example, in case of effector cells, effector cells bound with target cells via a synapse may be retained.

Without being bound by theory, as is understood the range of force that may break an aspecific cell-cell bond versus a specific cell-cell bond that forms a synapse differs. This difference can be to such an extent that the ranges of the required forces do not overlap. It is understood that some overlap may occur. Hence the force that is selected, as outline above, may allow for aspecific cell-cell bonds remaining and some specific cell-cell bonds that formed a synapse to break. In case there is substantially no overlap, a differential force can be selected, as outline above, which allows substantially for aspecific cell-cell bonds to break, while substantially retaining specific cell-cell bonds that formed a synapse. In case there is no overlap, and ranges are sufficiently far apart, a differential force may be selected, as outline above, which allows for aspecific cell-cell bonds to break while retaining specific cell-cell bonds that formed a synapse. As also outlined herein, the portion of cell-cell bonds that remains after exerting a force and has a synapse, can be determined by determining the presence or absence of a marker associated with synapse formation. This way, when selecting a force, *e.g.* in a scenario without comparing with control cells, which may be optional, the optimal force can be selected based on the cellular avidity scores involving synapse markers. Likewise the same principles can be applied for further cells of interest and target cells.

In one embodiment, after the step of applying a force away from attached cells in the methods of the invention, the cells are resuspended and a subsequent differential force is applied such that formed aspecific cell-cell bonds are broken. In another embodiment, in methods of the invention wherein either the target cells or cells of interest (*e.g.* effector cells) are attached to a surface, this attachment is optional, and, in the step of applying the force instead a differential force is applied. In yet another embodiment, in methods of the invention wherein the cells of interest are effector cells, either the target cells or effector cells are attached to a surface, this attachment is optional, and, in the step of applying the force instead a differential force is applied, wherein the differential force is such that cells that are bound via a synapse remain bound to each other and formed aspecific cell-cell bonds are broken.

### Examples

### Example 1

### Materials and methods

### Primary cells and cell lines

Untransduced or FMC63 CAR transduced Jurkat effector cells were obtained from Creative Biolabs, while Raji and NALM6 (ATCC) cells were used as target cells. All the cell lines were cultured with RPMI+Glutamax supplemented with 10% heat inactivated fetal bovine serum and Penicillin-Streptomycin, unless otherwise indicated.

### Avidity measurement

z-Movi^{®} chips (obtained and as available from LUMICKS (<<https://lumicks.com/products/z-Movi^{®}-cell-interaction-studies/>>), with channel dimensions of 7x2x0.1 mm and made of glass) were coated the day before performing the experiment with Poly-L-Lysine (#P4707-50ML, Sigma) diluted 1:5 in PBS for 10 minutes. The following morning the target cells were seeded in serum-free culture medium, incubated for 1 h at 37°C, and the medium was replaced with RPMI complete medium. The target cells were incubated for 1.5 h before starting the cellular avidity experiment. The effector cells were stained with CellTrace^{™} Far Red dye (Thermo Fisher Scientific) at 1 µM for 15 minutes in PBS at 37°C, then resuspended at 10 million/mL in complete medium and used for the avidity experiments. The effector cells (*i.e.* cells of interest) were introduced in the target cell-seeded flow cell, incubated for 5 minutes, and then different force ramps were applied throughout 1 or 2.5 minutes using z-Movi^{®} operated on the Oceon^{®} V1.4 software which was customized to allow control of force duration (time) and control of force increase (*i.e.* pN/s). The whole procedure in general can be performed in about 2-3 hours.

### Varying force

The acoustic force on the effector cells was applied as a linear ramp from 0 to 1000 or from 0 to 3000 pN over 1 or 2.5 minutes.

### Results

We hypothesized that different strength of binding defines the interaction between the transduced effector cell-target cell and untransduced effector cell-target cell (control cell-target cell) and that changing the way we apply the force could improve the positive/negative window, while preserving the transduced-target cell binding and reducing the aspecific binding of the untransduced sample.

We tested this hypothesis by monitoring the percentage of effector cells bound to the target cells after application of different acoustic force ranges. A linear force ramp applied over the course of 2.5 minutes to 1000 pN on Jurkat effector cells layered on NALM6 target cells resulted in a ~47% difference in percentage of bound cells at the end of the ramp between the positive and negative untransduced control. Increasing the target end force to 3000 pN, while keeping constant the force ramp time to 2.5 minutes enhanced the difference in percentage of bound cells at the end of the ramp to ~68%, thus considerably improving the positive/negative window of the assay (see Figure 2).

We next repeated the experiment using as target Raji cells, a cell line displaying high levels of aspecific binding of the untransduced sample. While a force ramp of 2.5 minutes to 1000 pN resulted in a difference of ~26%, a ramp with an end force to 3000 pN gave a difference between the positive and negative sample of ~52% (see Figure 3).

We also subjected the target cell monolayer to multiple force ramps at different end forces, and qualitatively evaluated the viability and the confluency of the target cell monolayer after staining with a Trypan Blue solution at the end of the measurements and could not observe differences across three different cell lines between chips acquired with the lower or higher end force ramps (see Figure 4). This observation ruled out that the decrease in background binding observed and shown in Figures 2 and 3 was due to a decrease of the target cell monolayer viability or integrity.

### Example 2

### Materials and methods

### Primary cells and cell lines

FMC63 CAR transduced Jurkat effector cells were obtained from Creative Biolabs, while NALM6 (ATCC) cells were used as target cells. All the cell lines were cultured with RPMI+Glutamax supplemented with 10% heat inactivated fetal bovine serum and Penicillin-Streptomycin, unless otherwise indicated.

### Avidity measurement

A glass coverslip was treated with a 12 M HCl solution for 10 minutes at room temperature for cleaning purpose, washed with water and thereafter contacted with a 1 M aqueous sodium hydroxide solution for 10 minutes at room temperature for activation purposes. Next, a mini well was created on the glass coverslip using Kapton tape (donut shaped) essentially as described in Yang et al., Nat. Commun. 2016, 7:11026 DOI: 10.1038/ncomms11026.

The created mini well was coated with poly-L-Lysine (Sigma) diluted 1:5 in PBS for 10 minutes at room temperature.

The target cells were seeded in serum-free culture medium, incubated for 0.5 h at 37°C, and the medium was replaced with RPMI complete medium. The target cells were incubated for 0.5 h before starting the cellular avidity experiment.

The effector cells were stained with CellTrace^{™} Far Red dye (Thermo Fisher Scientific) at 1 µM for 15 minutes in PBS at 37°C, then resuspended at 1 million/mL in complete medium and used for the avidity experiments. The effector cells (*i.e.* cells of interest) were introduced in the target cell-seeded mini well, incubated for 5 minutes, and then a second glass coverslip was placed on top of the mini well, thereby sealing it.

The sealed mini well was introduced in a Centrifuge Force Microscope (CFM) (see Yang and Wong, Methods Mol. Biol. 2018, 1665:353-366) capable of detecting fluorescent cells. The CFM was used to collect avidity data by applying force using two linear force profiles (see below), recording camera-acquired images during centrifugation (see Yang and Wong, Methods Mol. Biol. 2018, 1665:353-366), counting cells and calculating % of bound cells at four force points (see below).

### Varying force

The centrifugal force on the effector cells was applied as a fast linear force ramp (6.6pN/s) or a slow linear force ramp (3.3pN/s)). The % of bound cells was calculated at four force points: 100pN, 200pN, 300pN and 400pN.

### Results

The results are shown in Table 1. They demonstrate that applying a fast linear centrifugal force ramp resulted in a 17-22% increased difference in percentage of bound effector cells at various force points within a force ramp when comparing a fast force ramp with a slow force ramp. A fast force ramp enhanced the difference in percentage of bound cells at various force points within the ramp compared to a slow force ramp, thus considerably improving the positive/negative window of the avidity assay when using centrifugal forces.

**Table 1. Percentage of bound cells at various centrifugal force ramps.**

| | Bound cells at 100pN (in %) | Bound cells at 200pN (in %) | Bound cells at 300pN (in %) | Bound cells at 400pN (in %) |
|---|---|---|---|---|
| CAR T-cells (Fast linear force ramp (6.6pN/s)) | 78 | 77 | 75 | 70 |
| CAR T-cells (slow linear force ramp (3.3pN/s)) | 61 | 58 | 53 | 48 |

## Claims

1. Method for selecting and/or sorting cells of interest, comprising the steps of:
i) providing a selected force by means of a method comprising the steps of:
a) providing target cells (2) attached to a surface (1);
b) providing cells of interest (7) and control cells of the cells of interest (7);
c) contacting the control cells with the target cells to allow the control cells to interact with the target cells;
d) applying a first force (6) to the control cells, wherein the force is in a direction away from the target cells;
e) determine control cells that have detached and/or remain attached in step d) and provide a cellular avidity score for the control cells;
f) perform steps c), d) and e) with the cells of interest instead of the control cells;
g) repeat steps c)-f) by applying a second force;
h) optionally, perform steps c, d) and e) with one or more further forces;
i) select the force to be applied from the first, second and optional further forces by comparing, with the same force applied, the cellular avidity scores as determined in step e) with the cells of interest and of the control cells;
ii) providing target cells attached to a surface;
iii) providing cells of interest;
iv) incubating the cells of interest with the target cells attached to the surface in the presence of agent(s) capable of modulating the cellular avidity between the cell of interest and the target cell;
v) applying the selected force on the cells of interest and subsequently selecting and/or sorting cells of interest that have detached and/or that remain attached to the target cells.

2. The method in accordance with claim 1, wherein the applied force is a force ramp, preferably a linear force ramp.

3. The method in accordance with claim 2, wherein when the applied force is a force ramp, the different forces applied as a first, second, and optional further force, include providing for each force ramp a force loading rate and an end force.

4. The method in accordance with any one of claims 1-3, wherein the applied force is an acoustic force, a shear flow force or a centrifugal force.

5. The method in accordance with any one of claims 1-4, wherein the contacting step c) of the control cells and of the cells of interest, and subsequent steps d) and e) are performed separately or wherein these steps are performed with the control cells and cells of interest combined.

6. The method in accordance with any one of claims 5, wherein in the selection step of a force, from the selected force ramp the force rate is selected and the end force, or the force rate is selected and a lower force end force.

7. The method in accordance with any one of claims 1-6, wherein the target cells are attached to a glass surface, preferably a glass surface in a chip.

8. The method in accordance with any one of claims 1-7 wherein the target cells are attached to the surface as a monolayer.

9. The method in accordance with any one of claims 1-8, wherein the cells of interest are effector cells.

10. The method in accordance with claim 9, wherein of the effector cells bound with the target cells after the step of applying the force the presence of a marker associated with synapse formation is determined.

11. The method in accordance with claim 10, wherein cellular avidity scores are determined based on the number of effector cells with the marker associated with synapse formation.

12. The method in accordance with any one of claims 1-11, wherein after the step of applying a force the cells are resuspended and a differential force is applied such that formed aspecific cell-cell bonds are broken.

13. The method in accordance with any one of claims 1-12, wherein said target cells are optionally attached to a surface, and in the step of applying the force instead a differential force is applied.

14. The method in accordance with claim 13, wherein the cells of interest are effector cells, and the differential force is such that cells that are bound via a synapse remain bound to each other and formed aspecific cell-cell bonds are broken.

## Patentansprüche

1. Verfahren zum Auswählen und/oder Sortieren von Zellen von Interesse, umfassend die Schritte:
i) Bereitstellen einer ausgewählten Kraft durch ein Verfahren umfassend die Schritte:
a) Bereitstellen von Zielzellen (2), die an eine Oberfläche (1) gebunden sind;
b) Bereitstellen von Zellen von Interesse (7) und Kontrollzellen der Zellen von Interesse (7);
c) Inkontaktbringen der Kontrollzellen mit den Zielzellen, um zu ermöglichen, dass die Kontrollzellen mit den Zielzellen wechselwirken;
d) Aufbringen einer ersten Kraft (6) auf die Kontrollzellen, wobei die Kraft in einer Richtung weg von den Zielzellen gerichtet ist;
e) Bestimmen von Kontrollzellen, die sich bei Schritt d) abgelöst haben und/oder gebunden bleiben, und Bereitstellen eines zellulären Aviditätswert für die Kontrollzellen;
f) Durchführen der Schritte c), d) und e) mit den Zellen von Interesse anstelle der Kontrollzellen;
g) Wiederholen der Schritte c)-f) durch Aufbringen einer zweiten Kraft;
h) gegebenenfalls Durchführen der Schritte c, d) und e) mit einer oder mehreren weiteren Kräften;
i) Auswählen der aufzubringenden Kraft aus den ersten, zweiten und optionalen weiteren Kräften durch Vergleichen der wie bei Schritt e) bestimmten zellulären Aviditätsbewertungen mit den Zellen von Interesse und den Kontrollzellen für die gleiche aufgebrachte Kraft;
ii) Bereitstellen von an eine Oberfläche gebundenen Zielzellen;
iii) Bereitstellen von Zellen von Interesse;
iv) Inkubieren der Zellen von Interesse mit den an die Oberfläche gebundenen Zielzellen in Gegenwart eines oder mehrerer Mittel, die fähig sind, die zelluläre Avidität zwischen der Zelle von Interesse und der Zielzelle zu modulieren;
v) Aufbringen der ausgewählten Kraft auf die Zellen von Interesse und anschließend Auswählen und/oder Sortieren von Zellen von Interesse, die sich gelöst haben und/oder an den Zielzellen gebunden bleiben.

2. Verfahren nach Anspruch 1, wobei die aufgebrachte Kraft eine Kraftrampe, vorzugsweise eine lineare Kraftrampe, ist.

3. Verfahren nach Anspruch 2, wobei, wenn die aufgebrachte Kraft eine Kraftrampe ist, die verschiedenen Kräfte, die als eine erste, zweite und optionale weitere Kraft aufgebracht werden, Bereitstellen einer Kraftbelastungsrate und einer Endkraft für jede Kraftrampe enthalten.

4. Verfahren nach einem der Ansprüche 1-3, wobei die aufgebrachte Kraft eine akustische Kraft, eine Scherströmungskraft oder eine Zentrifugalkraft ist.

5. Verfahren nach einem der Ansprüche 1-4, wobei der Schritt des Inkontaktbringens c) der Kontrollzellen und der Zellen von Interesse und nachfolgende Schritte d) und e) getrennt durchgeführt werden oder wobei diese Schritte mit den Kontrollzellen und Zellen von Interesse kombiniert durchgeführt werden.

6. Verfahren nach einem der Ansprüche 5, wobei bei dem Schritt des Auswählens einer Kraft aus der ausgewählten Kraftrampe die Kraftrate und die Endkraft ausgewählt wird oder die Kraftrate und eine Endkraft mit geringerer Kraft ausgewählt wird.

7. Verfahren nach einem der Ansprüche 1-6, wobei die Zielzellen an einer Glasoberfläche, vorzugsweise einer Glasoberfläche in einem Chip, gebunden sind.

8. Verfahren nach einem der Ansprüche 1-7, wobei die Zielzellen als eine Monoschicht an die Oberfläche gebunden sind.

9. Verfahren nach einem der Ansprüche 1-8, wobei die Zellen von Interesse Effektorzellen sind.

10. Verfahren nach Anspruch 9, wobei von den Effektorzellen, die nach dem Schritt des Aufbringens der Kraft an die Zielzellen gebunden sind, das Vorhandensein eines Markers bestimmt wird, der mit Synapsenbildung verbunden ist.

11. Verfahren nach Anspruch 10, wobei zelluläre Aviditätsbewertungen auf der Grundlage der Anzahl von Effektorzellen mit dem mit Synapsenbildung verbundenen Marker bestimmt werden.

12. Verfahren nach einem der Ansprüche 1-11, wobei nach dem Schritt des Aufbringens einer Kraft die Zellen resuspendiert werden und eine Differenzkraft derart aufgebracht wird, dass gebildete aspezifische Zellen-Zellen-Bindungen gebrochen werden.

13. Verfahren nach einem der Ansprüche 1-12, wobei die Zielzellen gegebenenfalls an eine Oberfläche gebunden sind und bei dem Schritt des Aufbringens der Kraft stattdessen eine Differenzkraft aufgebracht wird.

14. Verfahren nach Anspruch 13, wobei die Zellen von Interesse Effektorzellen sind und die Differenzkraft so ist, dass Zellen, die über eine Synapse gebunden sind, aneinander gebunden bleiben, und gebildete aspezifische Zellen-Zellen-Bindungen gebrochen werden.

## Revendications

1. Procédé de sélection et/ou de tri de cellules d'intérêt, comprenant les étapes de :
i) fourniture d'une force sélectionnée au moyen d'un procédé comprenant les étapes de :
a) fourniture de cellules cibles (2) fixées à une surface (1) ;
b) fourniture de cellules d'intérêt (7) et de cellules témoins des cellules d'intérêt (7) ;
c) mise en contact des cellules témoins avec les cellules cibles afin de permettre aux cellules témoins d'interagir avec les cellules cibles ;
d) application d'une première force (6) aux cellules témoins, dans lequel la force est dans une direction s'éloignant des cellules cibles ;
e) détermination des cellules témoins qui se sont détachées et/ou qui restent fixées à l'étape d) et fourniture d'un score d'avidité cellulaire pour les cellules témoins ;
f) réalisation des étapes c), d) et e) avec les cellules d'intérêt au lieu des cellules témoins ;
g) répétition des étapes c) à f) en appliquant une seconde force ;
h) éventuellement, réalisation des étapes c), d) et e) avec une ou plusieurs forces supplémentaires ;
i) sélection de la force à appliquer parmi la première force, la seconde force et les forces supplémentaires facultatives en comparant, avec la même force appliquée, les scores d'avidité cellulaire tels que déterminés à l'étape e) avec les cellules d'intérêt et les cellules témoins ;
ii) fourniture de cellules cibles fixées à une surface ;
iii) fourniture de cellules d'intérêt ;
iv) incubation des cellules d'intérêt avec les cellules cibles fixées à la surface en présence d'un ou d'agent(s) capable(s) de moduler l'avidité cellulaire entre la cellule d'intérêt et la cellule cible ;
v) application de la force sélectionnée sur les cellules d'intérêt, puis sélection et/ou tri des cellules d'intérêt qui se sont détachées et/ou qui restent fixées aux cellules cibles.

2. Procédé selon la revendication 1, dans lequel la force appliquée est une rampe de force, de préférence une rampe de force linéaire.

3. Procédé selon la revendication 2, dans lequel, lorsque la force appliquée est une rampe de force, les différentes forces appliquées en tant que première force, seconde force et force supplémentaire facultative comprennent la fourniture, pour chaque rampe de force, d'un taux de chargement de force et d'une force finale.

4. Procédé selon l'une quelconque des revendications 1 à 3, dans lequel la force appliquée est une force acoustique, une force d'écoulement en cisaillement ou une force centrifuge.

5. Procédé selon l'une quelconque des revendications 1 à 4, dans lequel l'étape de mise en contact c) des cellules témoins et des cellules d'intérêt, et les étapes suivantes d) et e) sont réalisées séparément ou dans lequel ces étapes sont réalisées avec les cellules témoins et les cellules d'intérêt combinées.

6. Procédé selon la revendication 5, dans lequel, lors de l'étape de sélection d'une force, à partir de la rampe de force sélectionnée, le taux de chargement de force est sélectionné et la force finale, ou le taux de chargement de force est sélectionné et une force finale inférieure.

7. Procédé selon l'une quelconque des revendications 1 à 6, dans lequel les cellules cibles sont fixées à une surface en verre, de préférence une surface en verre dans une puce.

8. Procédé selon l'une quelconque des revendications 1 à 7, dans lequel les cellules cibles sont fixées à la surface sous forme de monocouche.

9. Procédé selon l'une quelconque des revendications 1 à 8, dans lequel les cellules d'intérêt sont des cellules effectrices.

10. Procédé selon la revendication 9, dans lequel, parmi les cellules effectrices liées aux cellules cibles après l'étape d'application de la force, la présence d'un marqueur associé à la formation de synapses est déterminée.

11. Procédé selon la revendication 10, dans lequel les scores d'avidité cellulaire sont déterminés sur la base du nombre de cellules effectrices avec le marqueur associé à la formation de synapses.

12. Procédé selon l'une quelconque des revendications 1 à 11, dans lequel, après l'étape d'application d'une force, les cellules sont remises en suspension et une force différentielle est appliquée de manière à rompre les liaisons cellule-cellule aspécifiques formées.

13. Procédé selon l'une quelconque des revendications 1 à 12, dans lequel lesdites cellules cibles sont éventuellement fixées à une surface, et dans l'étape d'application de la force, une force différentielle est plutôt appliquée.

14. Procédé selon la revendication 13, dans lequel les cellules d'intérêt sont des cellules effectrices, et la force différentielle est telle que les cellules qui sont liées par une synapse restent liées les unes aux autres et que des liaisons cellule-cellule aspécifiques sont rompues.
